# EUROPEAN PATENT SPECIFICATION

(11) **EP 2 502 563 B1**
(45) Date of publication and mention of the grant of the patent: **21.06.2017**
(21) Application number: 12160737.8
(22) Date of filing: 22.03.2012
(51) Int. Cl.: A61B 6/00

(54) **Multiplane medical imaging system**
Multiplanes System für medizinische Bildgebung
Système d'imagerie médicale multiplan

(30) Priority: 24.03.2011 FR 1152457
(43) Date of publication of application: 26.09.2012
(73) Proprietor: General Electric Company, Schenectady, NY 12345 (US)
(72) Inventor: BOUVIER, Bernard, 78530 Buc (FR)
(74) Representative: Bedford, Grant Richard

(56) References cited:
- EP-A1- 1 527 738
- US-A- 4 426 725
- US-A1- 2004 170 255
- US-A1- 2005 195 945

## Description

The invention relates in general to medical imaging systems and notably to multiplane medical imaging systems, that is to say capable of producing radiographic images in several planes and thus allowing the examination of an area of interest in several planes.

Imaging systems usually comprise an X-ray apparatus comprising an X-ray tube and an X-ray detector placed opposite the X-ray tube in a direction of emission of the X-rays. The tube and the detector are usually placed on two mutually opposite ends of an arm.

Such systems are used for angiographic examinations for diagnostic or interventional purposes.

During these examinations, it is necessary to produce radiographs by X-rays of an area of interest in the body of a patient. For this purpose, after the patient has been laid out on an examination table, the X-ray tube and the detector are brought to face the area to be radiographed.

In the prior art, there are several types of X-ray systems making it possible to produce radiographs.

Known first of all are the X-ray systems fixed to the floor, and in which the arm supporting the X-ray tube and the detector comprise several degrees of freedom making it possible to position the X-ray beam facing the area of interest.

This type of system however has a major drawback relating to the fact that the radiography need is necessary during only a limited time of the procedure. Meanwhile, it is access to the patient that must be given priority. The systems can therefore not be moved away from the examination table when they are not in use. In particular, the transfer and the installation of the patient on the examination table are hampered by the presence of this cumbersome system. It has therefore been proposed to mount the X-ray apparatus on a mobile device mounted on wheels driven by drive motors controlled automatically under the control of a navigation system. In this respect it is possible to refer to document FR 2 945 724.

Moreover, there are X-ray systems called "surgical mobile" units that can be moved manually. In this case, they are mounted on a carriage that contains a certain number of batteries used to supply the X-ray tube with power. This type of system is then not suitable for angiographic examinations because the necessary power delivered by the X-ray tube is no longer sufficient to obtain adequate image quality and, in particular, adequate contrast.

Moreover, this type of mobile X-ray system does not allow complex angulations because the diameter of the arm supporting the tube and the detector is not big enough. Similarly, these mobile X-ray systems do not achieve sufficient rotation speeds to allow good quality, three-dimensional image reconstructions. Finally, even though the weight of such a system is half as much as that of an X-ray apparatus designed for angiography, it remains very difficult to move because of its relatively large dimensions and of its weight, which can be up to 300 kg.

Finally, X-ray systems are known for angiography that are suspended from the ceiling and can be moved on guiderails via a mobile carriage driven for example with the aid of an electric motor.

When angiographic examinations are carried out, it may be necessary to use multiplane medical imaging systems, for example biplane systems, that are capable of forming images of a vessel in several planes in order to view the vessels in these different planes, most frequently perpendicular.

Biplane medical imaging systems therefore comprise two X-ray apparatuses each capable of forming an image in one plane. These apparatuses are for example mounted either on the floor or on the ceiling.

It is possible in this respect to refer to document US 2009/00 28 290 which describes various embodiments of a multiplane medical imaging system comprising, for example, a first X-ray apparatus mounted on a robot fixed to the floor, and a second X-ray apparatus supported by a second robot that can move relative to the floor or is mounted so as to slide on rails provided on the ceiling.

As can be conceived, in this type of medical imaging system, the X-ray apparatuses are relatively bulky so that, during the radiological examination, access to the patient laid out on an examination table is considerably limited.

This access limitation is notably due to the presence of the first X-ray apparatus that is fixed to the floor in the vicinity of the frontal zone of an examination or operating table on which the patient is laid out and which considerably limits access to the patient in this zone.

Moreover, the radiographic requirements for certain examinations or interventions are necessary only for a limited time of the procedure. Meanwhile, it is access to the patient or to the examination table that must have priority, so it is desirable that the X-ray apparatuses are both moved away from the table, for example during the transfer and the installation of the patient on the table.

Finally, in certain types of examination for interventional purposes, it may be essential to keep the examination table, itself mobile, in a fixed position in order to prevent moving the patient so that, in this case, the X-ray apparatuses of the multiplane imaging system are the only items to be moved and brought to face the area of interest to be radiographed.

Various known systems are also described, for example, in US 2005/0195945 and US 2004/0170255 which discloses an X-ray system comprising two mobile X-ray devices and a common control means for controlling said devices depending on their relative position. Here again, fixing the robot to the floor is likely to limit the ability to move the X-ray apparatus.

In view of the foregoing, there is therefore a need to have a multiplane medical imaging system and notably a biplane system that is capable of increasing the degrees of freedom of the X-ray apparatuses by increasing their ability for movement.

The subject of the invention is therefore, according to a first aspect, a multiplane medical imaging system as defined by the appended claims comprising a first X-ray apparatus and a second X-ray apparatus each comprising an X-ray tube.

According to a general feature of this system, the first and second X-ray apparatuses each comprise a mobile automatic device on which the X-ray tube and the detector are mounted in order to control the automatic movement of the first and second apparatuses.

By virtue of mounting the two apparatuses on mobile automatic devices, the movement capabilities of the X-ray apparatuses are considerably increased, which notably makes it possible to improve access to the examination table.

According to another feature, the first and second X-ray apparatuses comprise common control means suitable for controlling the mobile devices of the first and second apparatuses depending on their relative position.

It is therefore possible to move the apparatuses in a coordinated manner in particular in order to place them in an out-of-the-way waiting position relative to the examination table or in order to adjust the isocentre of the apparatuses, or else in order to clear away a zone for access to the patient, without risking a collision, taking account of their relative position.

According to another feature, since the X-ray tube and the detector are mounted on robot arms articulated about articulation axes, the system comprises means for measuring the position of the arms.

For example, the arms comprise sensors for measuring the position of the articulation axes.

According to yet another feature, the control means comprise means for controlling the mobile devices according to the position of obstacles, such as the examination table.

In one embodiment, the first X-ray apparatus is supported by a robot that can be moved on the floor of an examination room.

The second X-ray apparatus is, for its part, supported by a robot that can be moved on rails on the ceiling of the examination room.

A further subject of the invention, according to a second aspect, is a method for automatically moving a multiplane medical imaging system comprising a first X-ray apparatus and a second X-ray apparatus each comprising an X-ray tube and an X-ray detector, in which information relating to the position of the first and second apparatuses is acquired and the movement of mobile automatic devices on which the X-ray tube and the detector of each of the first and second X-ray apparatuses are mounted is controlled according to the said information.

In one embodiment, second information relating to the position of obstacles is acquired and the movement of the mobile devices is controlled according to the said second information.

According to this method, it is also possible to automatically move the X-ray apparatuses to an out-of-the-way waiting position between two image-taking phases.

For example, during a monoplane image-taking phase by means of one of the X-ray apparatuses, the other apparatus is moved into the out-of-the-way waiting position.

Various objects, features and advantages of the invention will appear on reading the following description, given only as a non-limiting example, and made with reference to the appended drawings in which:
Figure 1 is a schematic view of a biplane X-ray system in the out-of-the-way waiting position;
Figure 2 shows the system of Figure 1 during an examination phase;
Figure 3 shows the system of Figures 1 and 2 during the taking of a monoplane view; and
Figure 4 shows the system of Figures 1 and 2 during the taking of a biplane view and in a configuration in which one of the apparatuses is positioned on one side of an examination table.

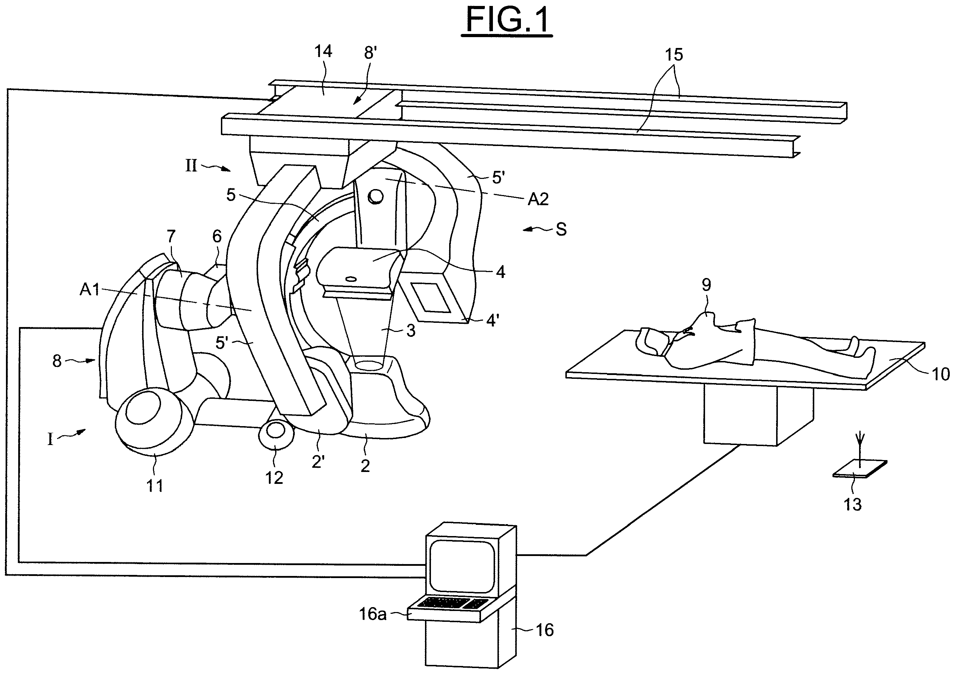

Figure 1 illustrates a biplane medical imaging system of the vascular type.

In the envisaged application, this imaging system is intended to be used for an angiographic examination and is in particular intended to provide views on two different planes of a vessel in order to view them in these two planes, which in this instance are perpendicular.

As can be seen, the imaging system S is therefore furnished with two X-ray apparatuses, referenced I and II, each ensuring a view in one plane.

These two apparatuses I and II are each mounted on a mobile robotized device and are then each capable of moving according to the examination phases.

The first X-ray apparatus I can be moved on the floor, while the second apparatus II can be moved on the ceiling of an examination or operating room.

With respect to the first apparatus I, the latter essentially comprises an X-ray tube 2, capable of emitting a beam 3 of X-rays in an emission direction, and an X-ray detector 4 placed at the two mutually opposite ends of an arm 5, in this instance in the form of an arch, so that the X-rays emitted by the tube 2 are incidental to the detector 4.

As can be seen, the arm 5 is mounted slidingly on a second arm 6 mounted so as to rotate on a fixed support 7, itself mounted on a mobile device 8.

Therefore, the support 7, the rotary arm 6 and the arm 5 are all articulated relative to one another about articulation axes, such as A1, so that the X-ray apparatus can be moved in three dimensions and thus take images of an organ to be examined at various angles of incidence.

During a radiography, the tube 2 and the detector 4 are brought to face an area of interest in the body 9 of a patient laid out on an examination table 10 so that, when the area of interest is interposed between the X-ray tube 2 and the detector 4, it is irradiated by the X-rays, and the detector 4 produces representative data of features of the interposed area of interest.

The mobile device 8 comprises, in the exemplary embodiment, a running system comprising, for example, two lateral drive and steering wheels 11 placed at the rear, two free front wheels 12, and means for driving the drive wheels comprising a steering motor coupled to a drive motor. The mobile device 8 is a robotized programmable device and is associated with a navigation system capable, for example, of communicating by radioelectric link with identification devices 13 placed in the operating room in order to allow the apparatus I to locate itself precisely in the room and, in particular, relative to the examination table 10.

The second X-ray apparatus II also comprises an X-ray tube 2' and an X-ray detector 4' placed opposite to the X-ray tube 2'.

The X-ray tube 2' and the detector 4' are each mounted on an articulated arm such as 5', the arms themselves each being mounted on a robotized mobile device 8'.

These arms are articulated about several articulation axes, such as A2, thus making it possible to adjust the position of the X-ray tube 2' and of the detector 4' in three dimensions relative to an area of interest.

The mobile device 8' in this instance comprises a support 14 provided with drive means and mounted slidingly on parallel longitudinal rails 15, themselves attached to the ceiling of the examination or operating room and having a first end positioned away from the examination table, corresponding to a parking position for the apparatus II and an opposite end situated facing the examination table, corresponding to an active position of the apparatus II.

The means for driving the support 14 are, for example made in the form of rollers driven by a drive motor on board the support or in the form of belts capable of moving the support depending on the examination phases.

Specifically, during a radiography, the tube 2' and the detector 4' are also brought to face an area of interest in the body 9 of the patient.

It is also possible to see in Figures 1 to 3 that the system S, and in particular the two X-ray apparatuses I and II, are connected to a common central processing unit 16, schematically represented, that is furnished with a control console 16a and duly programmed to control the movement of the two apparatuses I and II depending on the phases of an examination to be carried out.

In particular, the central processing unit 16 is furnished with storage means, of the data storage memory type, of the ROM, RAM, etc. type, incorporating one or more control algorithms capable of moving the X-ray apparatuses I and II either automatically, or under the control of the control console 16a, under the action of instructions entered manually by an operator.

As will be specified below, the central unit 16 also manages the movements of the apparatuses relative to the table 10.

In one embodiment, the central unit 16 also incorporates one or more navigation algorithms, stored in memory, in order to locate the apparatuses based on location information generated by detectors provided on the apparatus in order to communicate with the location devices.

Outside examination phases, the central processing unit controls the driving means of the mobile device 8 of the first X-ray apparatus I and of the mobile device 8' of the second X-ray apparatus so as to position the two apparatuses I and II in an out-of-the-way position (Figure 1) in order to clear the examination table 10 during the transfer and installation of the patient on the examination table.

This is also the case, during an intervention, when a radiological examination is not required.

This out-of-the-way position may correspond to a predetermined position stored in memory in the central unit 16 or to a control instruction entered by an operator by means of the console 16a.

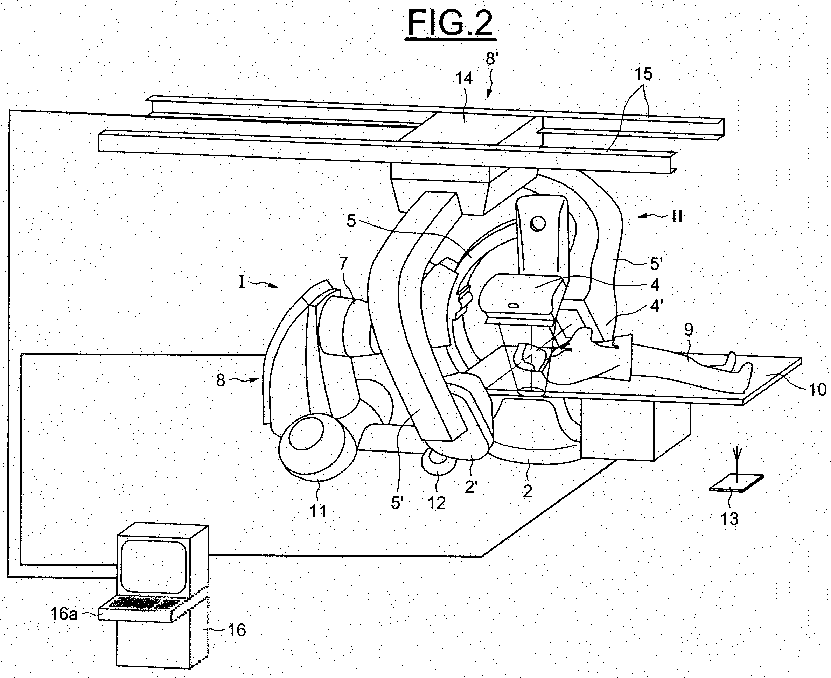

As can be seen in Figure 2, during a radiographic examination, the central processing unit 16 controls, either automatically, or under the control of the console 16a, the movement of the two X-ray apparatuses I and II so as to bring the X-ray tubes and the detectors to face the area of interest to be radiographed.

The movement of the apparatuses involves the use of the mobile devices 8 and 8' and the articulation of the arms in order to position the tubes and the detectors to face the area of interest.

During this phase, the isocentre of the axes of the two apparatuses, namely the meeting points of the X-rays emitted and received by each of the apparatuses, can be moved along the axis of the table without the patient having to be moved.

Moreover, during the radiographic examination, the first apparatus on the floor can be brought into various locations in an examination room, around the examination table, while controlling the positioning of the X-ray tube and of the detector in order to position them to face an area of interest.

The positions to which the apparatuses can be moved can be either positions programmed in memory in the central unit 16 or correspond to control instructions entered by the operator.

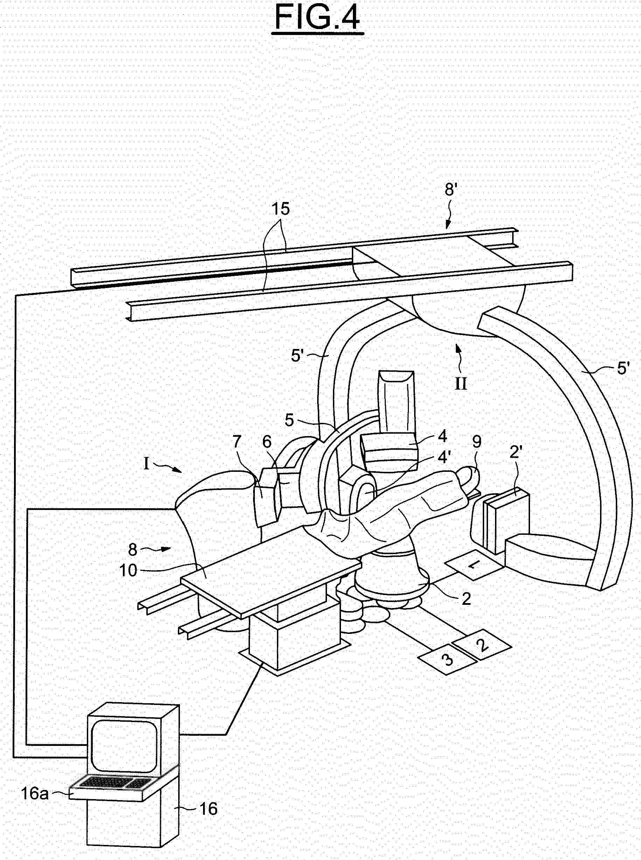

For example, as shown in Figure 4 which illustrates the imaging system of Figures 1 to 3 in a particular position of use, during a biplane radiography, the first apparatus on the floor can be moved to the vicinity of one of the longitudinal edges of the table while the tube and the detector are positioned to face an area of interest, in order to free up the frontal zone of the examination table, at which the head of the patient is situated.

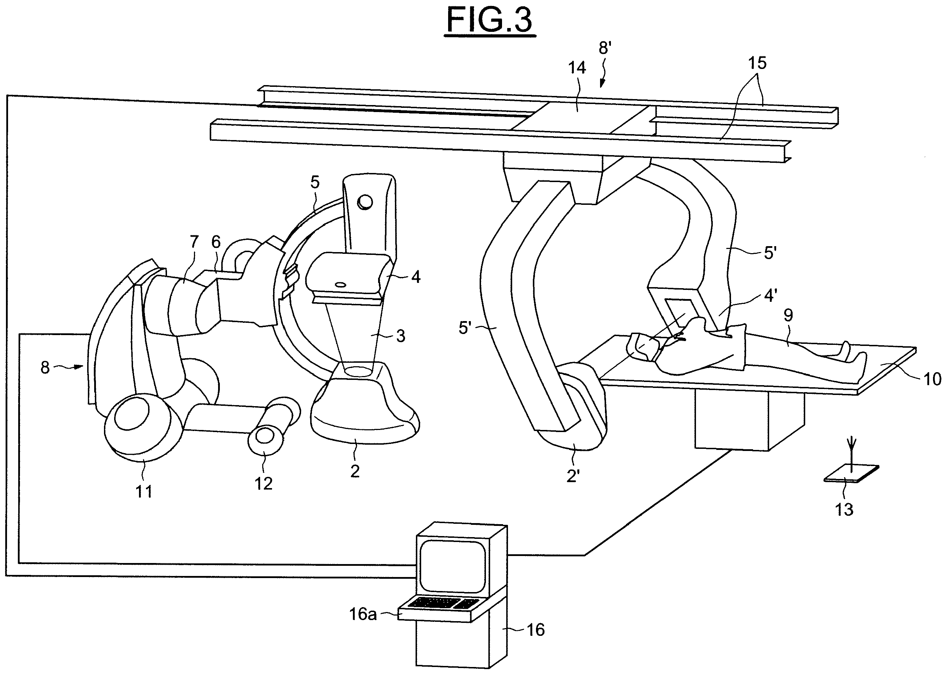

Finally it can be seen in Figure 3 that the central processing unit can cause the movement of one or other of the apparatuses into a position of use facing the area of interest and control the movement of the apparatus that is not in use into a retracted out-of-the-way position, thereby freeing up the space around the examination table 10.

It will be noted that the arms supporting the X-ray tubes and the detectors form robot arms comprising several articulation axes allowing them to be moved in three dimensions and to be positioned at various angles of incidence around an area of interest to be examined.

In order to prevent any risk of collision between the arms of the two apparatuses, the medical imaging system is furnished with means for measuring the position of the arms and, notably, of the articulation axes. These means are for example formed by a certain number of sensors installed in the arms and capable of measuring the angular position of the axes relative to a fixed reference point.

These various sensors are connected to the central processing unit 16 and thus provide the control programmes stored in memory with information relating to the position of all the arms of the X-ray apparatuses.

Therefore, the central processing unit 16 is kept informed at all times of the position of the arms and may allow or forbid a control instruction capable of causing a collision between the arms.

It will noted that the examination table 10 or, generally, any fixed or mobile obstacle in the examination room may also be provided with such sensors, connected to the central processing unit 16 in order likewise to inform the central processing unit 16 of the position of the examination table relative to the robot arm, and in consequence forbid or allow a control instruction that might cause a collision of the X-ray apparatuses with the table. Such sensors may be produced by any means, for example optical means, in order to deliver location information to the processing unit 16.

As can be understood, the embodiment of the invention that has just been described, which generally relates to a medical imaging system comprising a first X-ray apparatus and a second X-ray apparatus each comprising an X-ray tube and an X-ray detector in which the first and second X-ray apparatuses each comprise a mobile automatic device on which the X-ray tube and the detector are mounted in order to control the automatic movement of the first and second apparatuses, makes it possible to automatically move the two apparatuses into an out-of-the-way waiting position when they are not in use, to position one of the apparatuses in a working position and to keep the other apparatus in a waiting position, or else to adjust the isocentre of the two apparatuses without having to move the patient.

When one of the apparatuses can be automatically moved on the floor, certain zones around the examination table can be freed up, by moving this apparatus into another zone, while retaining the positioning of the X-ray tube and of the detector facing an area of interest.

Moreover, by virtue of the coupling of the apparatuses to a common central unit, and by virtue of controlling the X-ray apparatuses via the central unit according to their relative position, it is possible to produce images on several planes and to automatically move each of the apparatuses according to examination phases, in order to position them in an out-of-the-way waiting position or to bring them to face an area of interest and to do so while preventing any risk of collision.

## Claims

1. A multiplane medical imaging system for providing views of two different planes in an angiographic examination comprising a first X-ray apparatus (I) and a second X-ray apparatus (II) each comprising an X-ray tube (2, 2') and an X-ray detector X (4, 4'), wherein the first and second X-ray apparatus each comprise a mobile automatic device (8, 8') on which the X-ray tube and the detector are mounted in order to control the automatic movement of the first and second apparatuses, wherein the first X-ray apparatus (I) is supported by a robot (8) that can be moved on the floor of an examination room by means of wheels (11, 12), and the second X-ray apparatus (II) is supported by a robot (8') that can be moved on rails (15) on the ceiling of an examination room, said first X-ray apparatus (I) comprising a support (7), a rotary arm (6) and an arm (5) articulated with respect to one another such that the first X-ray apparatus (I) can be moved in three-dimensions, and the second X-ray apparatus (II) comprising a support (14) and an arm (5') articulated about several axes, wherein the first and second X-ray apparatuses comprise common control means (16) for controlling the mobile devices of the first and second apparatuses depending on their relative position and for controlling the mobile devices according to the position of obstacles, **characterised in that** the X-ray tubes (2, 2') and the detectors (4, 4') comprise means for measuring the position of the arms, and wherein the arms comprise sensors for measuring the position of the articulation axes, said first X-ray apparatus (I) being operable to communicate with identification devices (13) placed in an operating room in order to allow the first X-ray apparatus (I) to locate itself precisely relative to an examination table (10).

2. A method for automatically moving the multiplane medical imaging system according to claim 1, wherein information relating to the position of the first and second apparatuses is acquired and the movement of the mobile automatic devices (8, 8') on which the X-ray tube and the detector of each of the first and second X-ray apparatuses are mounted is controlled according to the said information.

3. The method according to Claim 2, wherein second information relating to the position of obstacles is acquired and the movement of the mobile devices is controlled according to the said second information.

4. The method according to Claim 2 or Claim 3, wherein the X-ray apparatus are moved automatically to an out-of-the-way waiting position between two image-taking phases.

5. The method according to any one of Claims 2 to 4, wherein, when a monoplane image is taken by means of one of the X-ray apparatuses, the other apparatus is moved into an out-of-the-way waiting position.

## Patentansprüche

1. Medizinisches Mehrebenenbildgebungssystem zum Vorsehen von Ansichten von zwei verschiedenen Ebenen bei einer Angiographieuntersuchung, umfassend eine erste Röntgenvorrichtung (I) und eine zweite Röntgenvorrichtung (II), die jede eine Röntgenröhre (2, 2') und einen Röntgendetektor (4, 4') umfassen, wobei die erste und zweite Röntgenvorrichtung jede ein mobiles automatisches Gerät (8, 8'), auf dem die Röntgenröhre und der Detektor angebracht sind, zum Steuern der automatischen Bewegung der ersten und zweiten Vorrichtung umfassen, wobei die erste Röntgenvorrichtung (I) durch einen Roboter (8) gestützt ist, der auf dem Fußboden eines Untersuchungszimmers mithilfe von Rädern (11, 12) bewegt werden kann, und die zweite Röntgenvorrichtung (II) durch einen Roboter (8') gestützt ist, der an Schienen (15) an der Decke eines Untersuchungszimmers bewegt werden kann, wobei die erste Röntgenvorrichtung (I) eine Stütze (7), einen Dreharm (6) und einen Arm (5) umfasst, die derart in Bezug zueinander gelenkig verbunden sein können, dass die erste Röntgenvorrichtung (I) in drei Dimensionen bewegt werden kann, und die zweite Röntgenvorrichtung (II) eine Stütze (14) und einen Arm (5') umfasst, der um mehrere Achsen gelenkig ist, wobei die erste und zweite Röntgenvorrichtung gemeinsame Steuermittel (16) zum Steuern der mobilen Geräte der ersten und zweiten Vorrichtung abhängig von ihrer relativen Position und zum Steuern der mobilen Geräte gemäß der Position von Hindernissen umfassen, **dadurch gekennzeichnet, dass**
die Röntgenröhren (2, 2') und die Detektoren (4, 4') Mittel zum Messen der Position der Arme umfassen, und wobei die Arme Sensoren zum Messen der Gelenkachsen umfassen, wobei die erste Röntgenvorrichtung (I) zum Kommunizieren mit Identifizierungsgeräten (13), die in einem Operationszimmer angeordnet sind, betriebsfähig ist, um zu ermöglichen, dass sich die erste Röntgenvorrichtung (I) präzise bezüglich eines Untersuchungstischs (10) anordnet.

2. Verfahren zum automatischen Bewegen des medizinischen Mehrebenenbildgebungssystems nach Anspruch 1, wobei Information bezüglich der Position der ersten und zweiten Vorrichtung erfasst wird und die Bewegung der mobilen automatischen Geräte (8, 8'), an denen die Röntgenröhre und der Detektor von jeder der ersten und zweiten Röntgenvorrichtung angebracht sind, gemäß der Information gesteuert wird.

3. Verfahren nach Anspruch 2, wobei zweite Information bezüglich der Position von Hindernissen erfasst wird und die Bewegung der mobilen Geräte gemäß der zweiten Information gesteuert wird.

4. Verfahren nach einem der Ansprüche 2 oder 3, wobei die Röntgenvorrichtungen automatisch zwischen zwei Bildaufnahmephasen in eine Warteposition abseits bewegt werden.

5. Verfahren nach einem der Ansprüche 2 bis 4, wobei, wenn ein Bild mit einer Ebene mittels einer der Röntgenvorrichtungen aufgenommen wird, die andere Vorrichtung in die Warteposition abseits bewegt wird.

## Revendications

1. Système d'imagerie médicale multiplan pour fournir des vues de deux plans différents dans un examen d'angiographie comprenant un premier appareil à rayons X (I) et un second appareil à rayons X (II), chacun comprenant un tube de rayons X (2, 2') et un détecteur de rayons X (4,4'), dans lequel le premier et le second appareil à rayons X comprennent chacun un dispositif automatique mobile (8, 8') sur lequel le tube de rayons X et le détecteur sont montés afin de commander le mouvement automatique du premier et du second appareil, dans lequel le premier appareil à rayons X (I) est supporté par un robot (8) qui peut être déplacé sur le plancher d'une salle d'examen au moyen de roues (11, 12) et le second appareil à rayons X (II) est supporté par robot (8') qui peut être déplacé sur des rails (15) au plafond d'une salle d'examen, ledit premier appareil à rayons X (I) comprenant un support (7), un bras rotatif (6) et un bras (5) articulés l'un par rapport à l'autre de sorte que le premier appareil à rayons X (I) puisse être déplacé dans trois dimensions, et le second appareil à rayons X (II) comprenant un support (14) et un bras (5') articulé autour de plusieurs axes, dans lequel le premier et le second appareil à rayons X comprennent des moyens de commande communs (16) pour commander les dispositifs mobiles du premier et du second appareil en fonction de leur position relative et pour commander les dispositifs mobiles en fonction de la position d'obstacles, **caractérisé en ce que** les tubes de rayons X (2, 2') et les détecteurs (4, 4') comprennent des moyens pour mesurer la position des bras et dans lequel les bras comprennent des capteurs pour mesurer la position des axes d'articulation, ledit premier appareil à rayons X (I) étant à même de communiquer avec des dispositifs d'identification (13) placés dans une salle d'opération afin de permettre au premier appareil à rayons X (I) de se localiser de manière précise par rapport à une table d'examen (10).

2. Procédé de déplacement automatique du système d'imagerie médicale multiplan selon la revendication 1, dans lequel des informations se rapportant à la position du premier et du second appareil sont acquises et le mouvement des dispositifs automatiques mobiles (8, 8') sur lesquels le tube de rayons X et le détecteur de chacun du premier et du second appareil à rayons X sont montés est commandé en fonction desdites informations.

3. Procédé selon la revendication 2, dans lequel des secondes informations se rapportant à la position d'obstacles sont acquises et le mouvement des dispositifs mobiles est commandé en fonction desdites secondes informations.

4. Procédé selon la revendication 2 ou la revendication 3, dans lequel les appareils à rayons X sont déplacés automatiquement en position d'attente hors trajet entre deux phases de prise d'images.

5. Procédé selon l'une quelconque des revendications 2 à 4, dans lequel, lorsqu'une image monoplan est prise au moyen de l'un des appareils à rayons X, l'autre appareil est déplacé en position d'attente hors trajet.
